# EUROPEAN PATENT APPLICATION

(11) **EP 3 254 732 A1**
(43) Date of publication of application: **13.12.2017**
(21) Application number: 16173745.7
(22) Date of filing: 09.06.2016
(51) Int. Cl.: A61P 1/04, A61K 39/00, C07K 16/12

(54) **IMMUNOPOTENTIATING COMPOSITIONS COMPRISING AN ANTI HELICOBACTER PYLORI ANTIBODY**

(71) Applicant: Sagabio Co., Ltd., Taipei City 10367 (TW)
(72) Inventor: LIAO, Kuang-Wen, Hsinchu City 300 (TW); JIAN, Ting-Yan, Hsinchu City 300 (TW)
(74) Representative: Winter, Brandl, Fürniss, Hübner, Röss, Kaiser, Polte - Partnerschaft mbB

(57) **Abstract**

The present invention provides a method for blocking immunosuppressive functions of pathogens, comprising: applying to an environment where the pathogens exist a composition that blocks immunosuppressive functions of the pathogens. The immunosuppressive functions are provided by an immunosuppressive substance secreted or produced by the pathogens. The composition includes a function inhibitor that comprises an antibody identifying the immunosuppressive substance or a fragment thereof. Use of the function inhibitor composition is also disclosed.

## Description

### FIELD OF THE INVENTION

The present invention relates to a method for blocking the immunosuppressive functions induced by a pathogen and use of the method, especially to a method for blocking the immunosuppressive functions provided by an immunosuppressive substance secreted or produced by the pathogen, to enhance the immune system of host of the pathogen, and use of the method.

### BACKGROUND OF THE INVENTION

*Helicobacter pylori* (*H. pylori*) is a Gram-negative bacterium that infects half of the adult population worldwide. The chronic inflammation triggered by *H. pylori* can lead to variable outcomes, such as peptic ulcers and gastric cancer, depending on the degree and extent of gastritis so caused. Although a predominant Th1-polarized mucosal immune response is activated in the host, the immune response is not sufficient to mount protective immunity against *H. pylori,* resulted in chronic infections and development of gastric pathologies in certain patient. Previous studies have revealed that *H. pylori* lysates can inhibit mitogen-induced T-cell proliferation, indicating that there are certain factors in the lysate relating to immunosuppressive activities. Such factors attenuate the T-cell activity, independent of the bacterial virulence genes CagA and VacA. Several mechanisms have been proposed to explain how *H. pylori* directly or indirectly suppresses T-cell-mediated immunity: *H. pylori* could inhibit T-cell proliferation and TCR expression by arginase, stimulate the release of the inhibitory cytokine TGF-β, interfere with invariant chain-dependent antigen presentation via VacA, negatively regulate the functions of DC via CagA phosphorylation, or suppress phagocytosis by professional phagocytes via VirB7 and VirB 11.

Despite the possible involvement of the above-mentioned mechanisms, regulatory T-cells (Treg cells) are currently considered the main regulatory components in the inhibition of T-cell activity and in the balance of inflammation and bacterial persistence. In 2003, CD4⁺CD25⁺ T-cells were reported to be involved in *H. pylori*-induced immunopathology and colonization. Further investigations showed that host Treg cells are crucial in protecting an *H. pylori*-infected host against excessive gastric inflammation and disease syndromes, while at the same time promote bacterial colonization at the gastric and duodenal mucosa. Moreover, the expression of B7-H1 by gastric epithelial cells promotes the development of CD4⁺CD25⁺FoxP3⁺Treg cells following *H. pylori* exposure, which indicates that this pathogen promotes the induction of host Treg cells. Subsequent studies examined the functions of these *H. pylori*-induced Treg cells and showed that they can suppress the activity or induce the anergy of *H. pylori*-specific effector T cells. In addition, *H. pylori*-induced gastritis is associated with a recruitment of naturally occurring FoxP3⁺ Treg cells that correlate with the degree of bacterial colonization and mucosal TGF-β1 expression. Collectively, these findings indicate that host Treg responses induced by *H. pylori* infection are important regulators of the immune response to *H. pylori* and are involved in the pathogenesis of *H. pylori*-related diseases.

*H. pylori* heat shock protein 60 (HpHSP60) can induce the expression of proinflammatory cytokines and TGF-β1 in monocytes. HpHSP60 has been reported to be expressed in the bacterial cell wall, associated with urease, and can function as an adhesive molecule for gastric epithelial cells. In addition, the administration of an anti-HSP60 antibody was found to interfere with the growth of *H. pylori.* Therefore, HpHSP60 is not only an essential factor for the viability of *H. pylori* but is also an important product that facilitates colonization of the human stomach. However, many studies have shown that HpHSP60 acts as a potent immunogen, leading to the strong induction of proinflammatory cytokines, such as TNF-α, IL-8, and IL-6. These cytokines determine inflammation at the site of infection, and such HpHSP60-induced inflammation might have the potential to promote processes of malignant tumorigenesis, including angiogenesis and metastasis. HpHSP60 is also an important virulence factor for *H. pylori* infection in a human host.

Taken together, the relationship between HpHSP60 and Treg cells is intriguing and deserves further investigations. However, most past researches focus on inflammation induced by HpHSP60. Very few researches talked about the relationship between HpHSP60 and the immunosuppressive reactions expressed in a host.

US patent No. 6,403,099 disclosed conjugated compounds comprising a heat shock protein and capsular oligosaccharide or polysaccharide. The compounds are capable of inducing formation of anti-polysaccharide antibodies. The heat shock protein includes *H. pylori* heat shock protein.

### SUMMARY OF THE INVENTION

The objective of this invention is to provide a method for blocking the immunosuppressive functions induced by a pathogen. Another objective of this invention is to provide a use for blocking the immunosuppressive functions induced by a pathogen.

The objective of this invention is also to provide a method for blocking the immunosuppressive functions provided by an immunosuppressive substance secreted or produced by the pathogen and use of the method.

The objective of this invention is also to provide a novel method for improving the immunity of a living body and use of the method.

According to this invention, certain pathogens are capable of suppressing the immunity of their hosts. Such immunosuppressive functions are especially provided by immunosuppressive substances secreted or produced by the pathogens. It is possible to block the immunosuppressive functions of the pathogens by shutting down functions of the immunosuppressive substances. Since the immunosuppressive functions have been shut down, the immunity activities of the host is not suppressed. The unsuppressed or increased immunity functions of the host are then capable of reducing or even eliminating the pathogens.

According to one aspect of this invention, a novel method for improving the immunity of a living body and its use in the preparation of pharmaceuticals for prevention, treatment of a disease are provided.

According to this invention, the method for increasing immunity of a living body comprises the step of applying directly or indirectly to a bacterial pathogen colonized internally or externally to the living body a composition that blocks an immunosuppressive function of the pathogen. In certain particular embodiments, the composition is applied to a live host organism attached to by the pathogens. In such and other embodiments, the immunosuppressive functions of the pathogens are provided by an immunosuppressive substance secreted or produced by the pathogens. Therefore, the composition blocking the immunosuppressive functions of pathogens includes a function inhibitor that blocks functions of the immunosuppressive substance. The function inhibitor comprises an antibody that identifies the immunosuppressive substance or a fragment thereof. The immunosuppressive substance may be a protein, in which case the fragment comprises a fragment amino acid sequence of the protein. The antibody may be one naturally generated by the immune response of a living body to the immunosuppressive substance. The antibody may be a humanized antibody of the antibody.

The composition may include an effective dose of the immunosuppressive substance and its pharmaceutically acceptable carrier or diluents. The composition may further include an adjuvant that increases immunestimulating effects.

The use of this invention includes using the immunosuppressive substance in the preparation of pharmaceuticals that stimulate or enhance immune response against the pathogens. Use of this invention may also include using the immunosuppressive substance in the preparation of pharmaceuticals that heal or prevents disease or symptom related to the existence or abnormal expression of the pathogens.

According to particular embodiments, a method for increasing the immunity of a living body comprises the step of applying to an environment where the pathogens exist a function inhibitor that blocks the functions of a heat shock protein secreted or produced by the pathogens. In such embodiments, the environment includes a live host organism attached by the pathogens. The pathogens may be *H. pylori* or any bacteria that attach to a surface of a live host organism.

In such and other embodiments, the function inhibitor includes an antibody that identifies the heat shock protein or a fragment thereof. The antibody may be one naturally generated by the immune response of a living body to the heat shock protein or a fragment amino acid sequence of the protein. The antibody may be a humanized antibody of the antibody.

The composition may include an effective dose of the function inhibitor and its pharmaceutically acceptable carrier or diluents. The composition may further include an adjuvant that increases immune-stimulating effects.

The use of this invention includes using the function inhibitor in the preparation of pharmaceuticals that stimulate or enhance immune response against the pathogen. The use of this invention may also include using the function inhibitor in the preparation of pharmaceuticals that treat or prevent disease or disorder relating to existence or abnormal expression of the pathogens.

### BRIEF DESCRIPTION OF THE DRAWINGS

Fig. 1 shows experimental results on effects of HpHSP60 on PBMC proliferation.
Fig. 2 shows experimental results on effects of HpHSP60 on T-cell proliferation.
Figs. 3A-3C respectively show experimental results on effects of HpHSP60 on PBMC cell cycles.
Fig. 4 shows experimental results on Treg cells *in vitro* induction by HpHSP60.
Fig. 5 shows experimental results on effects of HpHSP60 on Treg cell proliferation.
Fig. 6 shows experimental results on effects of HpHSP60-induced Treg cells on T-cell proliferation.
Fig. 7 shows experimental results on inhibition of *H. pylori in vivo* growth due blockage of HpHSP60 immunosuppressive functions.
Fig. 8 shows results of another experiment on inhibition of *H. pylori in vivo* growth due blockage of HpHSP60 immunosuppressive functions.
Fig. 9 shows experimental results on inhibition of Treg cells due blockage of HpHSP60 immunosuppressive functions.
Fig. 10 shows experimental results on active sequence in HpHSP60 that induces Treg cells growth.
Fig. 11 shows quantization of experimental results shown in Fig. 10.
Fig. 12 shows experimental results on a study of the immunological mechanism of anti-HpHSP60 antibodies.
Fig. 13 shows experimental results on effects of anti-HpHSP60 antibodies on Treg cells expressions in mice gastric mucosa.
Fig. 14 shows experimental results on effects of anti-HpHSP60 antibodies on IL-10 expressions in mice gastric mucosa.
Fig. 15 shows experimental results on recognition of HpHSP60 fragments by LHP-1 (9E4) antibody.
Fig. 16 shows results of further experiments on recognition of HpHSP60 fragments by LHP-1 (9E4) antibody.

### DETAILED DESCRIPTION OF THE INVENTION

Although it is not intended to limit the present by any theory, according to this invention, certain pathogens are capable of suppressing the immunity of their hosts, by secreting or producing immunosuppressive substances, so to proliferate or to cause disease to the host. Examples of such pathogens include *H. pylori* and other similar bacteria such as *Arcobacter suis, Tannerella forsythia, Porphyromonas gingivalis, Aggregatibacter actinomycetemcomitans* and *Helicobacter felis.* The inventors found that heat shock protein is one of such immunosuppressive substances. According to embodiments of this invention, *H. pylori* heat shock protein 60 (HpHSP60) is capable of reacting with monocytes to stimulate the production of immunosuppressive hormones, such as IL-10 and TGF-β, and induce the proliferation of Treg cells. As a result, the immunity of the host is suppressed, making the host unable to resist the chronic infections of *H. pylori.*

The present invention has developed a novel method to increase the immunity of hosts. A function inhibitor that blocks the functions of the immunosuppressive substances is used to shut down the functions of the immunosuppressive substances. Proliferation of Treg cells is thus effectively inhibited and immunosuppressive response of the host is eliminated.

A function inhibitor of this invention includes a substance that is capable of identifying the immunosuppressive substance or a fragment thereof and blocking functions of the immunosuppressive substance. When the immunosuppressive substance is a heat shock protein, the function inhibitor is an antibody capable of identifying the amino acid sequence, or a fragment thereof, of the heat shock protein.

The function inhibitor may be prepared in the form of a monoclonal antibody, to facilitate mass production. The function inhibitor may be prepared as a pharmaceutical composition, along with pharmaceutically acceptable carriers or diluent. The composition may further include an adjuvant that increases immunestimulating effects. One example of the adjuvant is the immunestimulating agent disclosed in the above-mentioned US patent No. 6,403,099. The pharmaceutical composition may be used as a drug that stimulates or improve immune response to the pathogens. It may also be used as a drug that treats or prevents a disease or disorder relating to the existence or abnormal expressions of the pathogens.

In the followings, certain examples will be described by referring to the drawings, in order to illustrate the method for and use of blocking immunosuppressive functions of pathogens of this invention. It is however appreciated that the scope of this invention is not limited to any of the embodiments described. For example, although in the detailed description the pathogenesis mechanism of *H. pylori* and function inhibitor for HpHSP60 are used as examples in the description of the invention, the heat shock protein of other bacteria, such as *Helicobacter felis* (known cause of chronic enteritis) and *Arcobacter suis* (known cause of periodontal disease), also include an identical fragment of HpHSP60, such as HSP60 101-200. Therefore, the method and use of this invention are also useful in these and other pathogenesis bacteria, and other pathogens that have similar pathogenesis mechanism.

### Embodiment 1: Cell culture and isolation of PBMC and T-cells

Human peripheral blood mononuclear cells (PBMCs) from healthy donors were isolated by density gradient centrifugation using Ficoll-Paque Plus (GE Healthcare, Uppsala, Sweden) and resuspended in RPMI-1640 with 10% inactivated fetal calf serum and 1% penicillin-streptomycin. For monocyte depletion, PBMCs were cultured in 10-cm dishes at a density of 10⁶/ml overnight for monocyte attachment. The suspended cells were then collected by centrifugation at 1500 rpm for 15 min. Total T-cells were isolated from PBMCs by negative selection using a magnetic sorting device (Miltenyi Biotec, MA, USA). Briefly, PBMCs were incubated with a cocktail of biotin-conjugated antibodies, followed by microbead-conjugated anti-biotin Abs for magnetic depletion. T-cells were eluted according to the manufacturer's protocols.

Embodiment 2: The effect of HpHSP60 on PBMC proliferation.

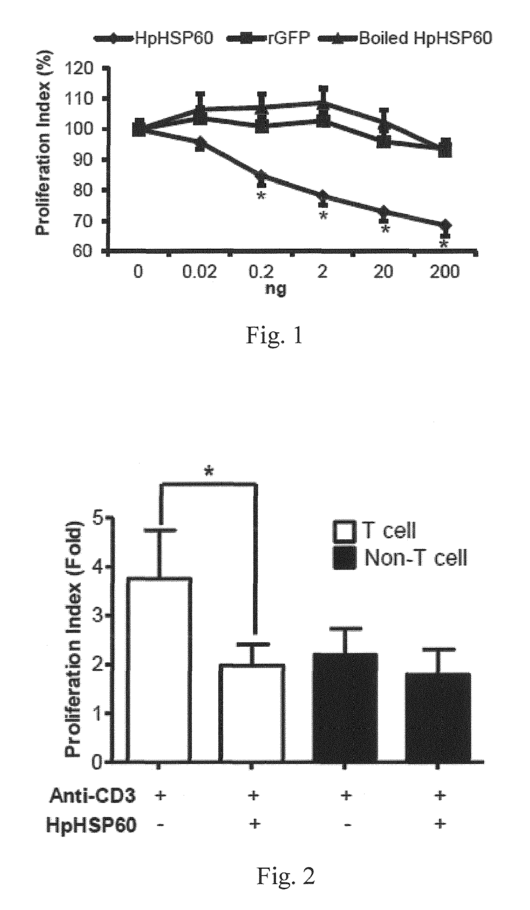

Proliferation of anti-CD3 mAb-stimulated PBMCs treated with HpHSP60, rGFP or boiled HpHSP60 at different doses was monitored by a cell proliferation assay. To measure cell proliferation, 0.2 ml of cells at 1x10⁶ cells/ml were seeded in each well of an anti-CD3 mAb-precoated 96-well microplate. Cell proliferation was determined by an MTT assay after 96 hours. Results are shown in Fig. 1: experimental results on effects of HpHSP60 on PBMC proliferation. Data shown therein are reported as the proliferation index.

The cell proliferation index was calculated as follows: Proliferation index (100%) = (OD₅₉₅ of the anti-CD3+HpHSP60-treated cells) / (OD₅₉₅ of the anti-CD3-treated cells)*100%. The results that differ significantly from the untreated group are indicated by * (p<0.05) (n=15).

In Fig. 1, (◆) shows proliferation of T-cells is inhibited, after HpHSP60 is added into the PBMC. (■) shows rGFP, being a control protein in this experimental system, does not influence T-cell proliferation. The results of this control unit show that not any protein is capable of inhibiting T-cell proliferation. (▲) represents boiled HpHSP60, which includes the sequence of HpHSP60, while its protein structure has been destructed. The results show that boiled HpHSP60 does not influence T-cell proliferation.

### Embodiment 3: Influence of HpHSP60 on T-cell proliferation in PBMC

After treatment with anti-CD3 mAb, PBMCs were treated with or without HpHSP60 (200 ng). T-cells or non-T-cells in PBMCs were identified by CD3 surface marker staining. Cell number was then calculated following a flow cytometer analysis.

For CD3 surface marker staining, cells were harvested and stained with 1 µg mouse anti-human CD3 IgG mAbs (OKT3), followed by 0.5 µg rabbit anti-mouse IgG-FITC secondary Abs (Biolegend, CA, USA). For FoxP3 intracellular staining, the cells were harvested and stained with mouse anti-human CD4-FITC mAbs (Biolegend, CA, USA) prior to fixing and permeabilization, followed by intracellular staining with mouse anti-human FoxP3-PE mAbs (BD Biosciences, MA, USA) according to the manufacturer's protocol. For the cell cycle assay, cells were harvested after 72 hours and 10⁶ cells were fixed with 70% ice-cold ethanol. DNA was stained with DNA staining buffer (5% Triton-X 100, 0.1 mg/ml RNase A, and 4 µg/ml propidium iodide) for 30 min at room temperature. Changes in the DNA content were then detected. Fluorescence was analyzed using a FACS flow cytometer (Becton Dickinson, Heidelberg, Germany) and CELLQuest Pro software (Becton Dickinson, Heidelberg, Germany). Results are shown in Fig. 2: experimental results on effects of HpHSP60 on T-cell proliferation.

In Fig. 2, the proliferation index is calculated as: proliferation index (fold) = (number of T- or non-T-cells in the anti-CD3/HpHSP60-treated group)/ (number of Tor non-T cells in the untreated control). A significant difference is indicated by *(*p*<0.05) (n=4). The results show that HpHSP60 is capable of inhibiting the proliferation of T-cells. In this figure, (□) represents T-cells in PBMC. (■) represents non-T-cells in PBMC. Clearly, what HpHSP60 inhibits is the proliferation of T-cells.

### Embodiment 4: Effects of HpHSP60 on the cell cycle

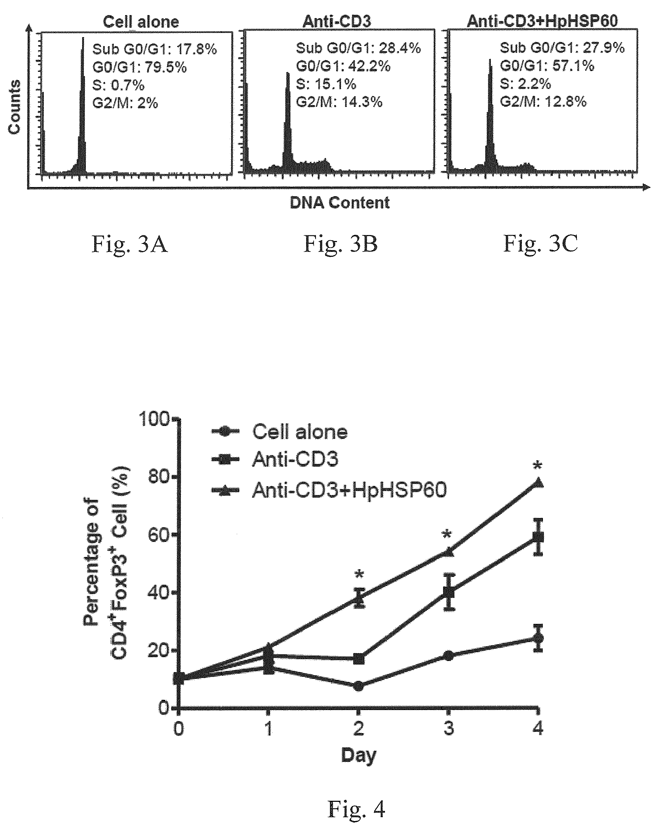

The effects of HpHSP60 on the cell cycle of PBMC were determined. From the PBMC products of Embodiment 3, PBMCs alone, CD3-activated PBMCs and PBMCs treated with anti-CD3 and HpHSP60 are obtained respectively. The percentages of cells in the sub-G1, G1, S, and G2/M phases are observed and presented in histogram plots, as shown in Fig. 3A-3C. Figs. 3A-3C respectively show experimental results on effects of HpHSP60 on PBMC cell cycles. The figures are representative of three replicates.

Figs. 3A-3C show that HpHSP60 inhibits the proliferation of T-cells, rather than causing them death. Fig. 3A shows T-cells without CD3 activation (Cell alone) remain in their dormant phases (G0/G). Fig. 3B shows that, after activation by CD3, the T-cells' growth was activated and the typical cell cycle graphics are formed. Figure 3C shows no substantial difference with Fig. 3B. PBMCs treated with anti-CD3 and HpHSP60 (Anti-CD3+HpHSP60) exhibit the same ratio at the sub G0/G1 phases (representing death of cells) as that of the Anti-CD3 group. The experimental results show that the role of HpHSP60 is to inhibit the growth of T cells, rather than resulting in death.

### Embodiment 5: Results of Treg cells in vitro induction by HpHSP60

The proportions of CD4⁺FoxP3⁺ cells in HpHSP60-treated PBMCs were measured over time. A significant difference compared to the anti-CD3 control is indicated by *(*p*<0.05) (n=5). The results are shown in Fig. 4: experimental results on Treg cells *in vitro* induction by HpHSP60.

Since CD4 and FoxP3 are markers for Treg cells, it is possible to identify HpHSP60's effects in T-cell growth from Fig. 4. In this figure, "(•) cell alone" expresses the original growth curve of T-cells. "(■) anti-CD3" expresses the growth curve of T-cells activated by CD3. "(▲) Anti-CD3 + HpHSP60" shows a significant proliferation of T-cells. The experimental results show that HpHSP60 is capable of enhancing Treg cell proliferation.

### Embodiment 6: HpHSP60 enhances Treg cell proliferation

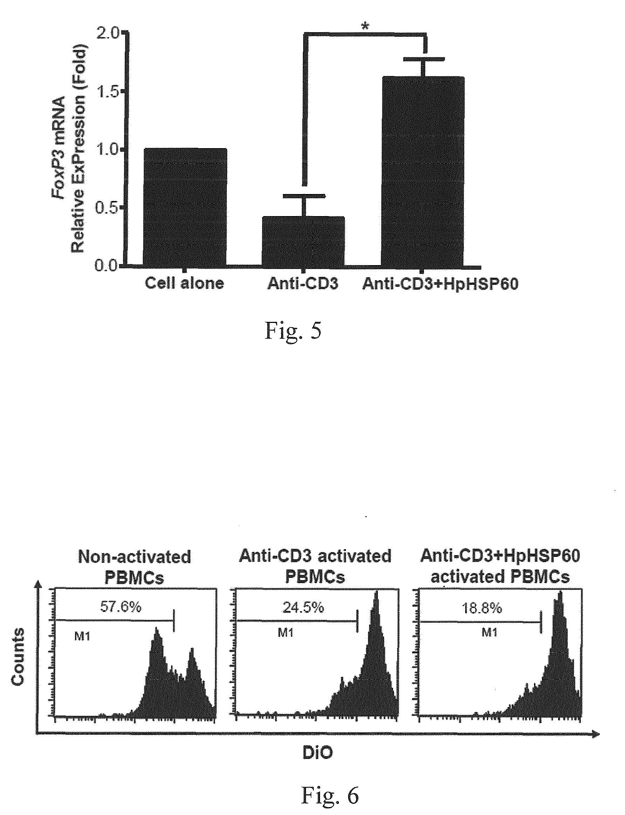

Following Embodiment 5, cells were harvested after 72 hours for total RNA isolation. Real-time PCR was used to measure the expression of *FoxP3* mRNA. A significant difference compared to the anti-CD3 control is indicated by *(*p*<0.05) (n=4). The results are shown in Fig. 5: experimental results on effects of HpHSP60 on Treg cell proliferation.

Since FoxP3 is marker for Treg cells. When Treg cells are activated, expression of FoxP3 also increases. The mRNA assay results of Fig. 5 show that, following the addition of HpHSP60, FoxP3 expression significantly increases. This experiment further supports the fact that adding HpHSP60 enhances Treg cell proliferation.

### Embodiment 7: The activity of HpHSP60-induced Treg cells on T-cell proliferation

Functional assay is used to measure the activity of HpHSP60-induced Treg cells on cell proliferation. The results are shown in Fig. 6. Fig. 6 shows experimental results on effects of HpHSP60-induced Treg cells on T-cell proliferation. Numbers on the histogram plots indicate the percentage of proliferative cells. The histogram plot is representative of three replicates.

The experimental results show that, when number of Treg cells increases, activities of T-cells are correspondingly inhibited. This proves that when HpHSP60 is added in the PBMC, activities of T-cells are inhibited, due to increase of Treg cells.

### Embodiment 8: Preparation of anti-HpHSP60 serum and HpHSP60 monoclonal antibodies

C3H/HeN mice were purchased from the National Laboratory Animal Breeding and Research Center, Taipei, Taiwan, and maintained in pathogen-free isolators. All food, water, caging, and bedding were sterilized before use. Male 5-week-old mice were i.v. injected with HpHSP60 to generate immunization reactions. After repeated boost of HpHSP60, blood of the mice is collected. Serum is isolated to obtain anti-HpHSP60 antibody containing serum, referred to as "anti-HpHSP60 serum." The products of this step are the polyclonal antibody.

The spleen cells of the mice were fused with mouse myeloma cells to form a hybridoma. The products are further screened by enzyme immunoassay (ELISA) to isolate specific antibodies.

The resulting cell lines were diluted and re-distributed into a cell culture plate with 96 wells. Calculate to ensure that each well contains only one cell. After the cells grow to form colonies, the colonies are again screened by ELISA to obtain specific antibodies. Monoclonal antibodies are thus obtained.

### Embodiment 9: Assessment of the eradication of H. pylori by blockage of HSP60 in vivo

C3H/HeN mice were purchased from the National Laboratory Animal Breeding and Research Center, Taipei, Taiwan, and maintained in pathogen-free isolators. All food, water, caging, and bedding were sterilized before use. Male 5-week-old mice were i.v. injected with 0.1 ml anti-HSP60 serum obtained from Embodiment 8 before *H. pylori* inoculation. At 24 hours after the anti-serum treatment, the mice were infected 0.5 mL live *H. pylori* (ATCC 15415 strain, approximately 10⁹ colony-forming units) in BHI broth by oral gavage twice within a 3-day period. After infection with *H. pylori* was established, the mice were then i.v. injected with 0.1 ml anti-HSP60 serum every 3 days.

At the 8th weeks after *H. pylori* inoculation, all of the mice were sacrificed aseptically and the intact stomachs were opened along the lesser curvature. Each stomach was dissected into two equal longitudinal specimens, containing the gastric body and antrum. The eradication of *H. pylori* was analyzed by *H. pylori* re-culture and immunohistochemistry staining for the expression of FoxP3.

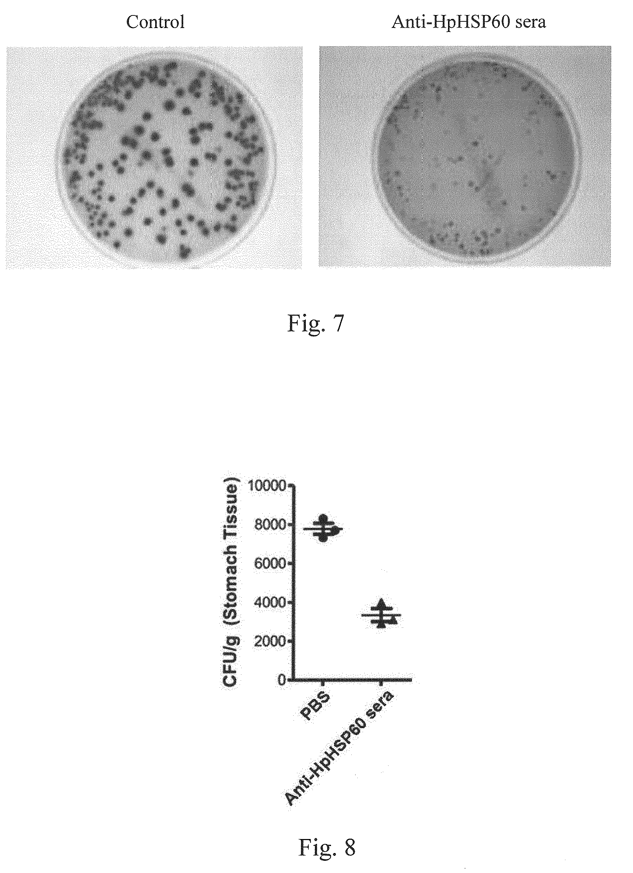

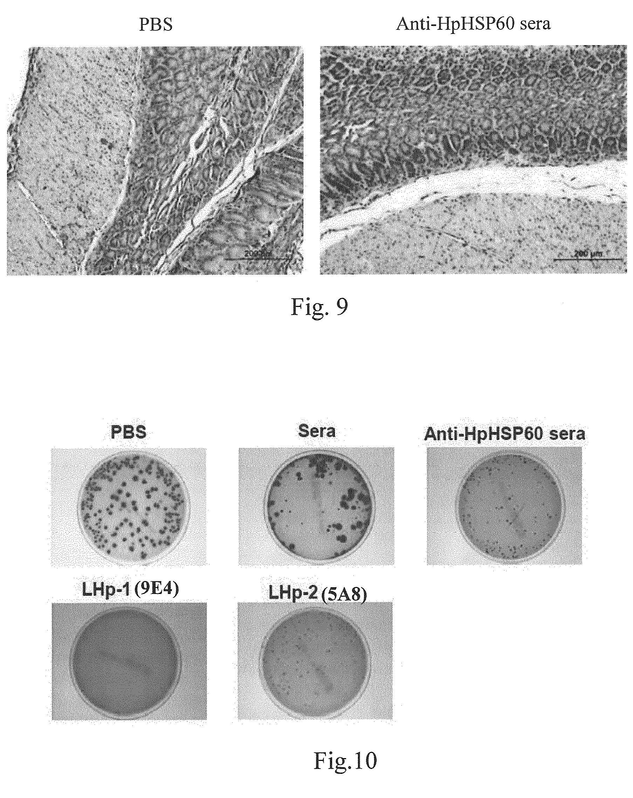

The results are presented as the mean ± SEM. The statistical significance was evaluated using the one-tailed Student's t-test; p < 0.05 was considered significant. The results are shown in Figs. 7, 8 and 9. Among them, Figs. 7 and 8 respectively show results of several experiments on inhibition of *H. pylori in vivo* growth due blockage of HpHSP60 immunosuppressive functions. Fig. 9 shows experimental results on inhibition ofTreg cells due blockage of HpHSP60 immunosuppressive functions.

Figs. 7 and 8 show that the anti-HpHSP60 serum significantly reduces the re-culture of *H. pylori* colonies from a gastric tissue lysate at the 8^{th} week after *H. pylori* inoculation. To determine the mechanism of colony decrease by antibodies, the expression ofTreg cells in *H. pylori*-infected gastric tissues was evaluated. Fig. 9 reveals that the anti-HpHSP60 serum treatments significantly reduce the expression of Treg cells in the gastric mucosa. These results indicate that chronic *H. pylori* infection is correlated with HpHSP60 and that the blockage of HpHSP60 can decrease *H. pylori* colonization and the generation ofTreg cells.

### Embodiment 10: Positions of Treg cell inducible sequence in HpHSP60

In order to allocate the position of active sequence in HpHSP60 that induces Treg cells, anti-HpHSP60 mAbs that recognize the full sequence or fragments of HpHSP60 are prepared. The method of Embodiment 9 is used. After 24-h treatment with the anti- HpHSP60 serum, the mice were infected by *H. pylori,* whereby infection of *H. pylori* Is established. The mice were then i.v. injected with 0.1 ml PBS, serum, anti-HSP60 serum, LHP-1 (9E4) mAb and LHP-2 (5A8) mAb, respectively, every 3 days. Mice were sacrificed after 8 weeks. The gastric wall was ground and the obtained gastric homogenates were incubated in *H. pylori* incubated isolation medium (EYE agar), to confirm *H. pylori* parasites in stomach. The results are shown in Figure 10: experimental results on active sequence in HpHSP60 that induces Treg cells growth.

As shown in Fig. 10, the red spots on the plate are colonies of *H. pylori.* This experiment reveals that, while anti-HpHSP60 serum inhibits growth of *H. pylori,* LHP-1 (9E4) antibody is capable of completely eliminating *H. pylori.*

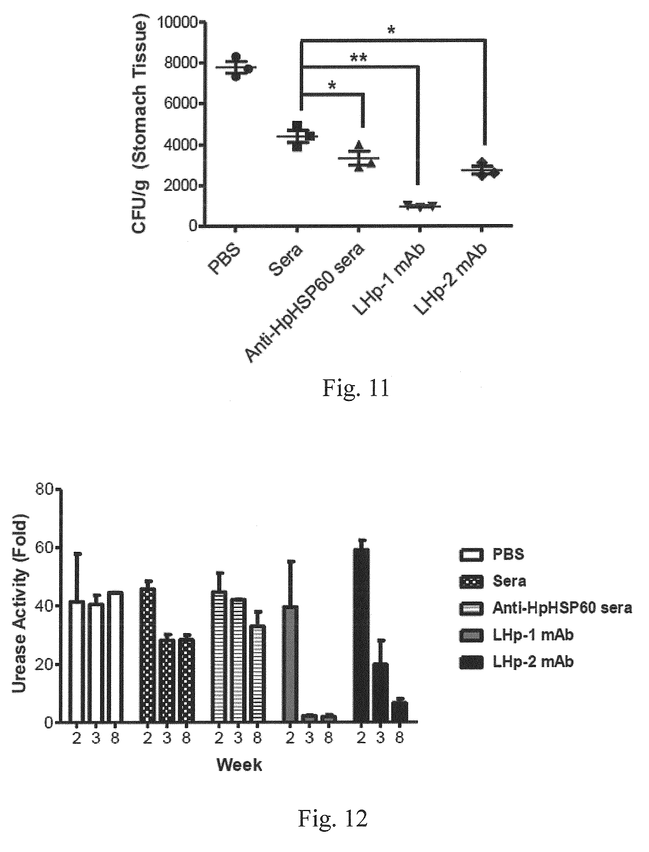

Number of *H. pylori* colonies (CFU) was determined by counting the red colonies on the EYE plate. A significant different is indicated by *(*p*<0.05). The results are shown in Fig. 11. Fig. 11 shows quantization of experimental results shown in Fig. 10. As shown in Fig. 11, *H. pylori* was completely eliminated after the LHP-1 (9E4) antibody was added.

### Embody 11: Immunological mechanisms of anti-HpHSP60 antibody

In order to understand immunological mechanisms of anti-HpHSP60 antibody, gastric urease activity of the mice according to Embodiment 10 at the 2^{nd}, 3^{rd} and 8^{th} week after *H. pylori* inoculation was measured. The urease activity is normalized to the gastric urease activity of the control mice (without *H. pylori* infection). The results are shown in Fig. 12: experimental results on a study of the immunological mechanism of anti-HpHSP60 antibodies. This figure shows that the LHP-1 (9E4) antibody inhibits growth of *H. pylori,* or even eliminates *H. pylori,* by inhibiting the activity of HpHSP60.

The LHP-1 (9E4) antibody was generated by using the amino acid sequence of positions 101 to 200 of HpHSP60 as antigen. Hybridomas including this antibody, LHP-1 (9E4), were deposited with the American Type Culture Collection (ATCC®), 10801 University Boulevard, Manassas, VA, 20110, USA, ATCC Designation: PTA-122900 (ATCC accession number: _). The date of deposit is 03/02/2016.

### Embodiment 12: Evaluation of expression of Treg cells in gastric mucosa

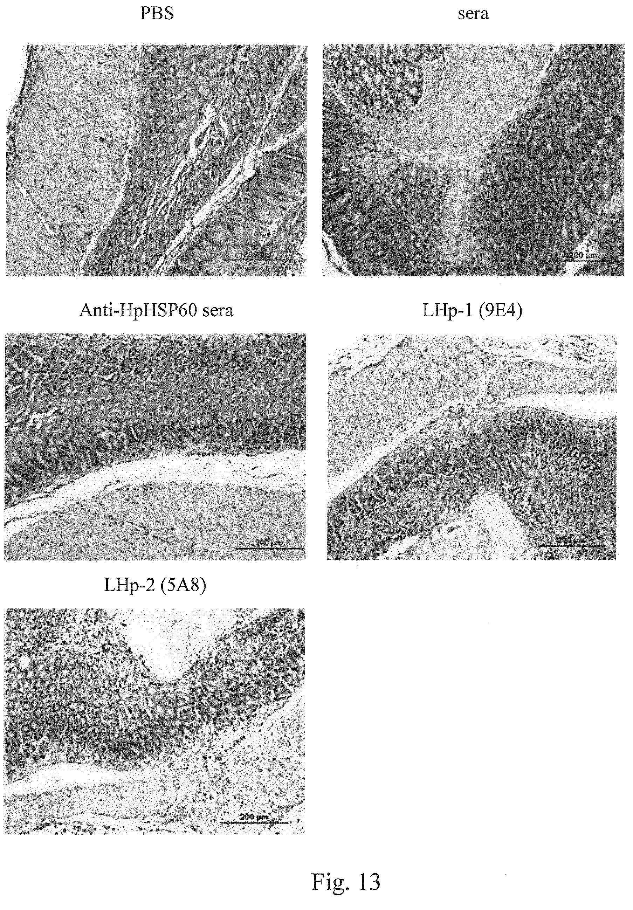

In order to understand anti-HpHSP60 antibody's effects in the expression of Treg cells in gastric mucosa, mouse stomachs obtained in Embodiment 10 were fixed with neutral buffered 10% formalin and embedded in paraffin. Five-micrometer sections were stained with H&E stain, followed by immunohistochemistry staining of FoxP3. The results are shown in Fig. 13. Fig. 13 shows experimental results on effects of anti-HpHSP60 antibodies on Treg cells expressions in mice gastric mucosa. In this figure, all pictures are representative of the mice sacrificed at the 8^{th} week (200 µm original magnification X100). The results show that no Treg expressions are observed in the mice gastric mucosa that was treated with LHP-1 (9E4) antibodies.

### Embodiment 13: Effects of HpHSP60 in expression of IL-10 in mice gastric mucosa.

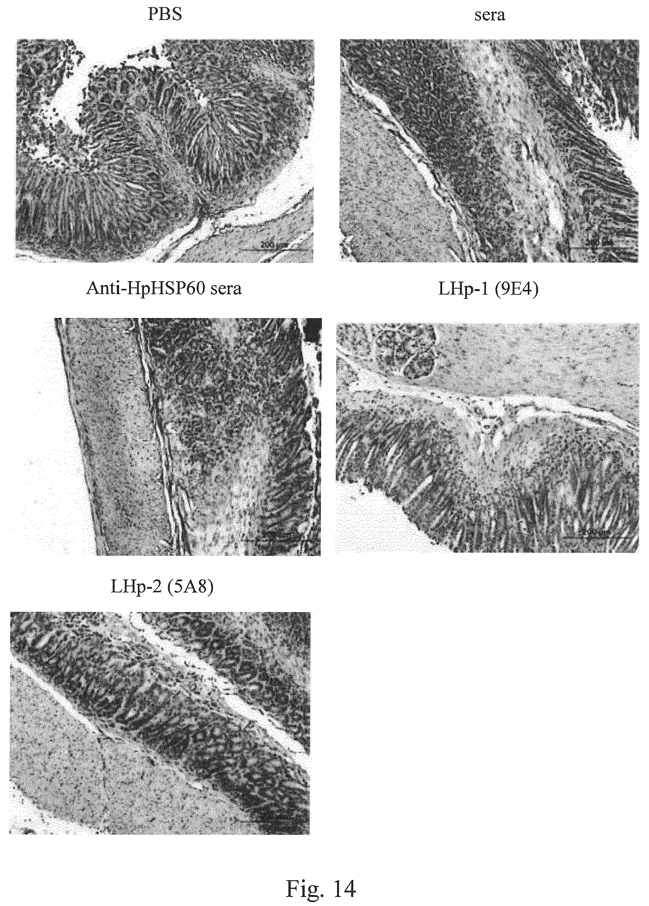

Mouse stomachs obtained in Embodiment 10 were fixed with neutral buffered 10% formalin and embedded in paraffin. Five-micrometer sections were stained with H&E stain, followed by immunohistochemistry staining of IL-10. The results are shown in Fig. 14. Fig. 14 shows experimental results on effects of anti-HpHSP60 antibodies on IL-10 expressions in mice gastric mucosa. In this figure, all pictures are representative of the mice sacrificed at the 8^{th} week (200 µm original magnification X100; 100 µm original magnification X200). The results show that no IL-10 expressions are observed in the mice gastric mucosa that was treated with LHP-1 (9E4) antibodies.

### Embodiment 14: Fragments of HpHSP60 identifiable by LHP-1 (9E4) antibody

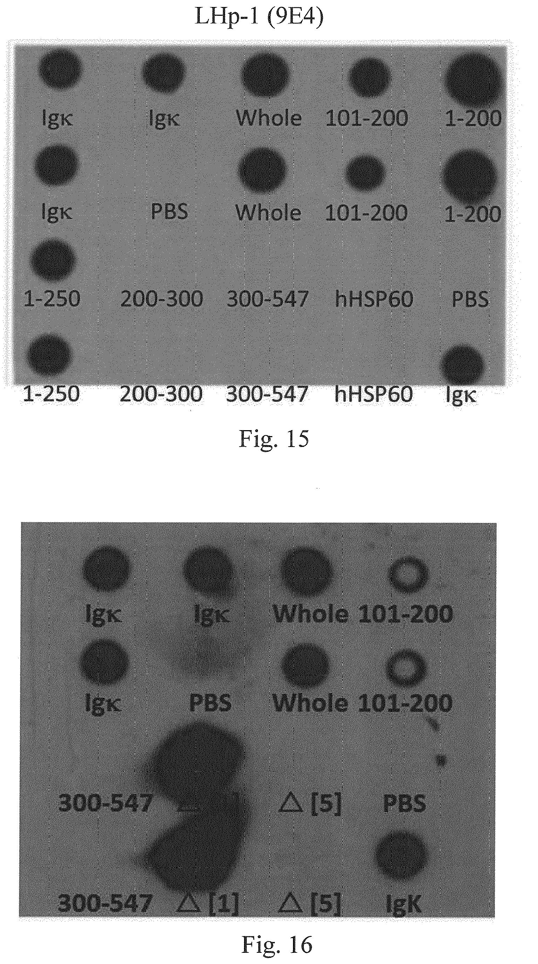

The LHP-1 (9E4) antibody is used to identify different lengths of fragments of HpHSP60, to determine fragments of HpHSP60 that can be recognized by the LHP-1 (9E4) antibody. The results are shown in Fig. 15. Fig. 15 shows experimental results on recognition of HpHSP60 fragments by LHP-1 (9E4) antibody. In this figure, dark spots represent positive identifications. Fragments being identified include the followings, while IgK is used as positive controls, since most mice mAbs are kappa type:
Whole - the full length of HpHSP60, i.e. positions 1-547.
1-200 - fragment including positions 1-200 of HpHSP60.
101-200 - fragment including positions 101-200 of HpHSP60.
1-250 - fragment including positions 1-250 of HpHSP60.
200-300 - fragment including positions 200-300 of HpHSP60.
300-547 - fragment including positions 300-547 of HpHSP60.

The results show that the HpHSP60 fragment identifiable by the LHP-1 (9E4) antibody is the sequence of positions 101-200, which amino acid sequence (Sequence No. 1) is:

### EGLRNITAGANPIEVKRGMDKAAEAIINELKKASKKVGGKEEITQVATISA NSDHNIGKLIADAMEKVGKDGVITVEEAKGIEDELDVVEGMQFDRGYLS

### Embodiment 15: Further limitation of HpHSP60 fragment identifiable by LHP-1 (9E4) antibody

Following the method of Embodiment 14, use the LHP-1 (9E4) antibody to identify different fragments with shorter lengths. The results are shown in Fig. 16. Fig. 16 shows results of further experiments on recognition of HpHSP60 fragments by LHP-1 (9E4) antibody. In this figure, dark spots represent positive identifications. Δ[1] represents positions 134-200 of HpHSP60, with positive results, while Δ[5] represents positions 101-168 of HpHSP60, with negative results. It can therefore be concluded that the HpHSP60 fragment identifiable by the LHP-1 (9E4) antibody includes amino acid positions 169-200 of HpHSP60, which are:
KDGVITVEEAKGIEDELDVVEGMQFDRGYLS (Sequence No. 2)
## Claims

1. A method for increasing immunity of a living body, comprising the step of applying directly or indirectly to a bacterial pathogen colonized internally or externally to the living body a composition that blocks an immunosuppressive function of the pathogen.

2. The method according to claim 1, wherein the composition is applied to an internal of the living body colonized by the pathogen.

3. The method according to claim 1, wherein the bacterial pathogen is one selected from the group consisted of *H. pylori, Arcobacter suis, Tannerella forsythia, Porphyromonas gingivalis, Aggregatibacter actinomycetemcomitans* and *Helicobacter felis.*

4. The method according to claim 1, wherein the immunosuppressive function of the bacterial pathogens is provided by an immunosuppressive substance secreted or produced by the pathogen and the composition blocking the immunosuppressive function of pathogen comprises a function inhibitor that blocks functions of the immunosuppressive substance.

5. The method according to claim 4, wherein the immunosuppressive substance secreted or produced by the pathogen comprises a heat shock protein or a fragment thereof.

6. The method according to claim 5, wherein the heat shock protein comprises heat shock protein 60 or a fragment thereof.

7. The method according to claim 6, wherein the heat shock protein 60 comprises a sequence of Sequence No. 1.

8. The method according to claim 4, wherein the function inhibitor comprises a chemical or an antibody that identifies the immunosuppressive substance or a fragment thereof.

9. The method according to claim 4, wherein the function inhibitor comprises a humanized product of an antibody that identifies the immunosuppressive substance or a fragment thereof.

10. The method according to claim 5, wherein the function inhibitor comprises an antibody that identifies the heat shock protein or a fragment thereof.

11. The method according to claim 10, wherein the function inhibitor comprises an antibody naturally generated by an immune response of the living body to the heat shock protein or a fragment thereof.

12. The method according to claim 4, wherein the composition comprises an effective dose of the immunosuppressive substance and its pharmaceutically acceptable carrier or diluents.

13. The method according to claim 12, wherein the composition further comprises an adjuvant that increases immunestimulating effects.

14. Use of the functional inhibitor according to any of claims 8-11 in the preparation of a pharmaceutical that stimulates or enhances immune response against the pathogen.

15. Use of the functional inhibitor according to any of claims 8-11 in the preparation of a pharmaceutical that heals or prevents a disease or symptom related to an existence or abnormal expression of the pathogen.

## Amended claims

### Amended claims in accordance with Rule 137(2) EPC.

1. Use of an inhibitor for an immunosuppressive function of a bacterial pathogen as a pharmaceutical for stimulating or enhancing an immune response against said bacterial pathogen, wherein said immunosuppressive function is provided by an immunosuppressive substance secreted or produced by the bacterial pathogen, said inhibitor comprising a composition containing an antibody that blocks the immunosuppressive functions of said immunosuppressive substance of the bacterial pathogen;
wherein said bacterial pathogen is selected from the group consisting of H. *pylori, Arcobacter suis, Tannerella forsythia, Porphyromonas gingivalis, Aggregatibacter actinomycetemcomitans* and *Helicobacter felis;*
wherein said immunosuppressive substance comprises a heat shock protein or a fragment thereof; and
wherein the inhibitor comprises an effective dose of the antibody and its pharmaceutically acceptable carrier or diluents.

2. Use according to claim 1, wherein said antibody is an antibody that identifies said immunosuppressive substance or a fragment thereof.

3. Use according to claim 1, wherein said antibody is a humanized antibody that identifies said immunosuppressive substance or a fragment thereof.

4. Use according to claim 1, wherein said antibody identifies said heat shock protein or a fragment thereof.

5. Use according to claim 1, wherein said antibody is a humanized antibody that identifies said heat shock protein or a fragment thereof.

6. Use according to claim 1, wherein said antibody is naturally generated by an immune response of a living body to the heat shock protein or a fragment thereof.

7. Use according to claim 1, wherein the composition further comprises an adjuvant that increases immunestimulating effects.

8. Use according to claim 1, wherein the heat shock protein comprises heat shock protein 60 or a fragment thereof.

9. Use according to claim 8, wherein the heat shock protein 60 comprises a sequence of Seq-ID No. 1.

10. Use of an inhibitor for an immunosuppressive function of a bacterial pathogen as a pharmaceutical for healing or preventing a disease or symptom related to an existence or abnormal expression of said bacterial pathogen, wherein said immunosuppressive function is provided by an immunosuppressive substance secreted or produced by the bacterial pathogen, said inhibitor comprising a composition containing an antibody that blocks the immunosuppressive functions of said immunosuppressive substance of the bacterial pathogen;
wherein said bacterial pathogen is selected from the group consisting of H. *pylori, Arcobacter suis, Tannerella forsythia, Porphyromonas gingivalis, Aggregatibacter actinomycetemcomitans* and *Helicobacter felis;*
wherein said immunosuppressive substance comprises a heat shock protein or a fragment thereof; and
wherein the inhibitor comprises an effective dose of the antibody and its pharmaceutically acceptable carrier or diluents


Sequence listing
<110> Sagabio Co., Ltd.
<120> METHOD FOR AND USE OF BLOCKING IMMUNOSUPPRESSIVE FUNCTIONS OF PATHOGENS
<130> SB3301P-EP
<160> 2
<210> 1
<211> 100
<212> PRT
<213> Helicobacter pylori
<400> 1
   Glu Gly Leu Arg Asn Ile Thr Ala Gly Ala Asn Pro Ile Glu Val Lys
   1               5                   10                  15
   Arg Gly Met Asp Lys Ala Ala Glu Ala Ile Ile Asn Glu Leu Lys Lys
               20                  25                  30
   Ala Ser Lys Lys Val Gly Gly Lys Glu Glu Ile Thr Gln Val Ala Thr
           35                  40                  45
   Ile Ser Ala Asn Ser Asp His Asn Ile Gly Lys Leu Ile Ala Asp Ala
       50                  55                  60
   Met Glu Lys Val Gly Lys Asp Gly Val Ile Thr Val Glu Glu Ala Lys
   65                  70                  75                  80
   Gly Ile Glu Asp Glu Leu Asp Val Val Glu Gly Met Gln Phe Asp Arg
                   85                  90                  95
   Gly Tyr Leu Ser
               100
<210> 2
<211> 31
<212> PRT
<213> Helicobacter pylori
<400> 2
   Lys Asp Gly Val Ile Thr Val Glu Glu Ala Lys Gly Ile Glu Asp Glu
   1               5                   10                  15
   Leu Asp Val Val Glu Gly Met Gln Phe Asp Arg Gly Tyr Leu Ser
               20                  25                  30